# EUROPEAN PATENT SPECIFICATION

(11) **EP 2 059 285 B1**
(45) Date of publication and mention of the grant of the patent: **29.12.2010**
(21) Application number: 07819986.6
(22) Date of filing: 28.08.2007
(51) Int. Cl.: A61M 5/24, A61M 5/31

(54) **A MEDICAL DELIVERY SYSTEM ADAPTED TO BE LOCKED AXIALLY AND UNLOCKED ROTATIONALLY**
AXIAL VERSCHLIESSBARES UND DURCH DREHUNG ZU ÖFFNENDES MEDIKAMENTENVERABREICHUNGSSYSTEM
SYSTÈME D'ADMINISTRATION MÉDICAL CONÇU POUR ÊTRE VERROUILLÉ PAR UN MOUVEMENT AXIAL ET DÉVERROUILLÉ PAR UN MOUVEMENT DE ROTATION

(30) Priority: 28.08.2006 EP 06017857
(43) Date of publication of application: 20.05.2009
(73) Proprietor: NOVO NORDISK A/S, 2880 Bagsværd (DK)
(72) Inventor: ELAHI, Ramin, Nateghi, DK-3330 Goerloese (DK); SMITH- TORRY, Jonas, DK-1622 Copenhagen V (DK); JAKOBSEN, Nikolaj, Eusebius, DK-2300 Valby (DK); HANSEN, Michael, Ejstrup, DK-5462 Morud (DK)
(86) International application number: PCT/EP2007/058932
(87) International publication number: WO 2008/025772

(56) References cited:
- EP-A- 0 897 728
- DE-U1- 20 110 690
- US-A- 5 611 783
- US-A1- 2004 238 776
- US-A1- 2006 153 693

## Description

### FIELD OF THE INVENTION

The present invention relates to a medical delivery system comprising a container and a dosing assembly. In particular the present invention relates to medical delivery system wherein the container is adapted to be locked to the dosing assembly through a non-rotational movement and unlocked from the dosing assembly through a non-translational movement. Moreover, the present invention relates to a container for use in the medical delivery system and to a dosing assembly for use in the medical delivery system.

### BACKGROUND OF THE INVENTION

Generally, in order to provide superior medication delivery devices which are likely to be well received by particular groups of patients, a greater diversity in drug delivery systems have been launched to the benefit of patients. As the number of commercially available delivery systems increase, numerous different types of medication holding cartridges or containers are distributed. Most of these types of containers differ in various aspects.

Each medicament container may be filled with a particular type of medicament selected from a large variety of different medicaments, but also different kinds of the same class of medicament (e.g. rapid or long acting insulin) and different concentrations of each particular medicament may be accommodated in the containers.

Moreover, different container volumes may be introduced in order to customize each container, and, thus, the delivery system to the needs of particular users. Variation of container volume may be provided by changing the length or diameter of the container. These modifications usually imply corresponding modifications of the dosing assembly of a medication delivery system, so as to provide a particular stroke of a driving element for expelling the medicament from the container or to provide optimal dosing precision. Further discrimination between different medicament containers may be occasioned by the design requirements for each particular delivery system, such as required sliding friction of the piston accommodated in the container.

In order to discriminate between a larger variety of available containers, numerous container coding and coupling systems have been developed. The following mechanical coding and coupling systems are known in the art:
US 5,611,783 relates to a pen shaped syringe comprising a distal part which may comprise an ampoule and a proximal part containing a dose setting and drive mechanism. The proximal and distal parts have interlocking bayonet coupling means. Protrusions may be provided to form a pattern ensuring that a certain distal part may only be used in connection with a certain proximal part.
WO 03/017915 A1 discloses a cartridge having a distal end provided with a mechanical coding. The mechanical coding has the form of a circular protrusion where the circular outer diameter is dedicated a specific concentration of insulin contained in the cartridge.
US 5,693,027 discloses a plastic top for adapting a standard cartridge to a chosen syringe. The plastic top may be provided with means for keyed engagement with corresponding means in a syringe to keep it unrotable when mounted with a cartridge in the syringe. In some types of syringes such keyed engagement between cartridge and syringe is further used to ensure that only a certain type of cartridge is used.
US 6,648,859 B2 discloses a drug cartridge assembly for use with a reuseable pen body assembly of a medication delivery pen. In order to eliminate cross-use the pen body assembly and the drug cartridge are keyed i.e. they may be threadedly engaged by corresponding threads and grooves, bayonet threads, and grooves, snap fits or a pair of lugs that mate in reverse Luer-Lock manner. The mating members are selected so as to prevent cross-use with other assemblies, e.g., the pitch of the threads may be angled so as to mate only with one another and not with other assemblies.

Yet another prior art system is described in DE 201 10 690 U1.

It is an object of a preferred embodiment of the present invention to provide an alternative to the known systems. Furthermore, it is an object of a preferred embodiment of the present invention to provide a medication delivery system with a large number of possible coding geometries.

Additionally, it is an object of a preferred embodiment of the present invention to provide a locking system wherein a container is locked to a dosing assembly through a first movement and unlocked through a second movement which is different from a reversed first movement.

Furthermore, it is an object of a preferred embodiment of the present invention to provide a coding system wherein the user experiences substantially the same operational fastening/coupling/locking movement when the container and dosing assembly of a predetermined medical delivery system are coupled/uncoupled (locked/unlocked) to each other regardless of the specific choice among sets of compatible container/dosing assemblies. Additionally, it is an object of a preferred embodiment of the present invention to provide a system having a large number of differently coded containers/dosing assemblies while simultaneously obtaining a rugged system where the possibility of mechanical failure is minimized

Furthermore, it is an object of a preferred embodiment of the present invention to provide an intuitive fastening mechanism for fastening the container to the dosing assembly.

### BRIEF DESCRIPTION OF THE INVENTION

In a FIRST aspect the present invention relates to a medical delivery system comprising:
- a container adapted to contain a medicament in a chamber defined by the container and a slidably arranged piston which is movable in a distal direction towards an outlet so as to reduce the volume of the chamber and expel the medicament;
- a dosing assembly adapted to be fastened to the container, so as to allow a driver of the dosing assembly to move the piston of the container in the distal direction;
- wherein the dosing assembly defines a first fastening means which during fastening of the container to the dosing assembly engages a second fastening means of the container, the first and/or second fastening means being shaped such that the container is adapted to be:
   - locked to the dosing assembly through a translational and non-rotational movement between one of the container and the dosing assembly and at least a part of the other one of the container and the dosing assembly, and
   - unlocked from the dosing assembly through a rotational and non-translational movement between one of the container and the dosing assembly and at least a part of the other one of the container and the dosing assembly.

In the context of the present invention the term "medical delivery system" shall be understood as any system capable of administering a medicament-containing flowable drug. Examples of medical delivery systems are infusion pump applications, dosers, pen-shaped dosers, motor-dosers, and automated syringes such as the AutoPen (TM).

The invention is applicable to all kinds of medicament delivery devices capable of delivering a medicament to a user from a container which is adapted to be coupled to a dosing assembly of the delivery device. The delivery device may include any delivery device for transcutaneous, subcutaneous, intravenous, intra muscular or pulmonary administration of a drug.

As used herein, the term "medicament" is meant to encompass any medicament-containing flowable drug capable of being passed through a delivery means such as a hollow needle in a controlled manner, such as a liquid, solution, gel or fine suspension. Representative medicaments includes pharmaceuticals such as peptides, proteins (e.g. insulin, insulin analogues and C-peptide), and hormones, biologically derived or active agents, hormonal and gene based agents, nutritional formulas and other substances in both solid (dispensed) or liquid form.

The chamber of the container may be defined by one or more sidewalls of the container and the slidably arranged piston. In most embodiments at least a part of the container is ring-shaped and defines a cylindrical cavity in which the piston is received. The distal end of the container may comprise a seal for penetration by a cannula so as to allow a medicament contained in the chamber to be expelled through the cannula. The distal end of the container may be adapted to be attached to a holder holding a cannula. As an example the distal end of the container may comprise a thread adapted to cooperate with a corresponding thread of the holder so as to allow the holder to be screwed onto the container.

The container may be provided as a cartridge holder adapted to receive a medicament filled cartridge, the cartridge having a slideably piston arranged internally and a piercable membrane for closing off a fluid outlet, where the cartridge is removably of fixedly accommodated in the cartridge holder. Alternatively, the container may be provided as a closed reservoir having a medicament fluid contacting the container wall sections and having a slideably arranged piston arranged internally and a piercable membrane for closing off a fluid outlet.

The outlet of the container may be adapted to cooperate with or be defined by a cannula or a needle or a needle hub or an infusion set, or any other fluid communicating conduit adapted to provide fluid access to a medicament accommodated in the container.

The driver of the dosing assembly may comprise a piston rod adapted to move the piston in the distal direction. The piston rod may comprise an element which is more rigid than the piston and is adapted to abut at least a part of and preferably most of the proximal facing surface of the piston whereby a force applied by the piston rod to the rigid element is applied to a larger area of the proximal surface of the piston than if the piston rod had engaged the piston directly.

In the context of the present invention the terms "depression" and "projection" are only used in connection with radially extending members/elements/means, and "indentation" and "protrusion" are only used in connection with axially extending members/elements/means. However, "depression" and "indentation" shall be seen as synonyms and "protrusion" and "projection" shall be seen as synonyms.

The container is adapted to be locked to the dosing assembly through a translational and non-rotational movement, such as a pure translational movement. Additionally, the container is adapted to be locked to the dosing assembly through a rotational and non-translational movement, such as a pure rotational movement.

In one embodiment, one of the container and the dosing assembly comprises one or more radially extending coding projections adapted to be received in corresponding radially extending coding depressions of the other one of the container and the dosing assembly, and the container is prevented from being locked to the dosing assembly unless the radially extending coding projections and the radially extending coding depressions define predetermined coding geometries.

The latter embodiment improves user safety as only predetermined containers may be attached to predetermined dosing assemblies. Thus, the dosing assembly may be designated to be used with a predetermined kind and/or concentration of a medicament and containers accommodating other concentrations or types of medicaments cannot be attached to the dosing assembly.

In one embodiment the radially extending projections may be moved axially into the radially extending depressions, such that upon further axial movement, the projections are moved out of the depressions, whereby the container and the dosing assembly are free or substantially free to be rotated relative to each other.

As the container and the dosing assembly are locked to each other through a translational and non-rotational movement, the radially extending coding projections and depressions need not be designed such that the depressions are wide enough circumferentially to allow the projections to be rotated about the longitudinal axis of the device, while being received in the depressions. This increases the total number of coding geometries (defined by one or more of the circumferential position, the radial extent, the circumferential extent, and the axial extent of the projections/depressions) as substantially no slack/gap between the projections and the depressions is needed.

The system may comprise a further level of user safety by comprising one or more axially extending protrusions adapted to be received in corresponding indentations in the other one of the container and the dosing assembly. Moreover, the container may be prevented from being locked to the dosing assembly unless the axially extending protrusions and the axially extending indentations define predetermined coding geometries.

In one embodiment the container and/or the dosing assembly may comprise a rotatable gripping member which may be operable from an outer surface of the device so as to allow the container and the dosing assembly to be locked and unlocked from each other. The rotatable gripping member may be adapted to be rotated about the longitudinal axis of the device in order to cause the first and second fastening means to disengage. In one embodiment the rotatable gripping member may be rotated less than one revolution in order to unlock or lock the container to the dosing assembly, such as less than 90 degrees, such as less than 45 degrees, such as less than 30 degrees, such as less than 15 degrees.

Furthermore, the rotatable gripping member may be ring-shaped and at least a part of the ring-shaped member may encirculate the container and/or the dosing assembly. A centre axis of the rotatable gripping member may coincide with a centre axis of the container and/or dosing assembly.

Alternatively, the rotatable gripping member does not encirculate the container and/or the dosing assembly and only a part of the outer surface of the medical delivering system is covered by the gripping member. As an example the rotatable gripping member may comprise a radially extending operator which when rotated about the longitudinal axis of the device causes the first and second fastening means to disengage.

In order to prevent accidental unlocking of the container from the dosing assembly, the rotatable gripping member may be axially movable between a locked and an unlocked position, such that when positioned in the locked position the rotatable gripping member is prevented from being rotated about the longitudinal axis of the device and when positioned in the unlocked position the rotatable gripping member is allowed to be rotated about the longitudinal axis of the device.

Additionally, the rotatable gripping member may be adapted to be maintained in the unlocked position by moving the rotatable gripping member axially relative to the dosing assembly and/or the container.

In one embodiment the container is locked to the dosing assembly through a translational and non-rotational movement, such as a pure translational movement, between the rotatable gripping member and the other one of the container and the dosing assembly. In the same embodiment, the container is unlocked from the dosing assembly through a rotational and non-translational movement, such as a pure rotational movement, between the rotatable gripping member and the other one of the container and the dosing assembly.

In a second embodiment the container is locked to the dosing assembly through a translational and non-rotational movement, such as a pure translational movement, between the container and the dosing assembly. In the same embodiment, the container is unlocked from the dosing assembly through a rotational and non-translational movement, such as a pure rotational movement, between the container and the dosing assembly. The rotational movement may be a relative rotation between the container and the dosing assembly of less than one revolution, such as less than 180 degrees, such as less than 90 degrees. In a specific embodiment the rotational movement is between 5 and 45 degrees, such as between 15 and 35 degrees, such as 15 degrees, such as 20 degrees, such as 25 degrees, such as 30 degrees.

In order to allow the translational locking and rotational unlocking, one of the first and second fastening means may define at least one radially extending fastening projection adapted to be received in a corresponding radially extending fastening depression of the other one of the first and second fastening means. Each fastening projection and depression may extend radially inward or outward during the coupling or uncoupling procedure. The container and the dosing assembly are locked for relative translational movement when the radially extending fastening projection(s) is/are received in the corresponding fastening depression(s). In particular embodiments, the radially extending fastening projection(s) or depression(s) is/are provided in-between two of the axially extending fastening protrusions or indentations forming the axially extending coding geometries.

In one embodiment one of the container and the dosing assembly encirculates the other one of the container and the dosing assembly when the container is fastened to the dosing assembly. In this embodiment the encirculating part may comprise a fastening projection extending radially inward and the encirculated part may comprise a fastening depression extending radially inward. Alternatively or as a supplement, the encirculated part may comprise a fastening projection extending radially outward and the encirculating part may comprise a fastening depression extending radially outward.

The container or dosing assembly may comprise one or more radially extending fastening projections such as one or more than one, two, three four or five. Additionally, the container or the dosing assembly may comprise one or more radially extending fastening depressions such as one or more than one, two, three, four or five. In one embodiment the radially extending fastening depressions extend through a sidewall of the container or dosing assembly such that the depression defines an opening on both an inner and outer surface of said sidewall. In another embodiment the depressions extend into the sidewall and defines only one opening in an inner or outer surface of the sidewall.

During locking of the container to the dosing assembly, the fastening projection may be biased into the depression so as to lock the container to the dosing assembly. In most embodiments the projection(s) is/are in a rest position when engaging the depression. Prior to being forced into the fastening depression, the fastening projection may be biased away from its rest position due to engagement between surfaces of the container and the dosing assembly.

In order to allow the projection to be biased away from its rest position the first and/or second fastening means may comprise an inclined surface. In one embodiment the inclined surface is provided on one or more of the fastening projection(s). The inclined surface may define a normal having a first component which is parallel with the centre axis of the medical delivery system and a second component extending in the radial direction of the device. When an axial force is applied to the inclined surface, the fastening projection is forced away from its rest position by being moved radially inward or outward depending on the design of the medical delivery system.

As an example the container may comprise a radially extending fastening projection having an inclined surface which when the container is moved towards the dosing assembly during fastening, engages a surface such as a rim of the dosing assembly. Upon further relative axial movement between the container and the dosing assembly, the radially extending fastening projection is moved away from the rest position due to the axial force applied to the inclined surface by the dosing assembly.

In order to allow the fastening projection to be moved radially, the radially extending fastening projection(s) or depression(s) may be provided in-between two of the axially extending fastening protrusions or indentations, whereby the radially extending fastening projection is provided on an axially extending protrusion. It will be appreciated, that the thinner the axially extending protrusion is radially and circumferentially the easier it will be to move the radially extending fastening projection radially.

When the radially extending fastening projection(s) is/are received in the corresponding fastening depressions, the container and the dosing assembly are locked for relative translational movement. In order to unlock the container from the dosing assembly, the fastening projections must be moved radially outward of the fastening depressions. This may be achieved rotating the dosing assembly and the container relative to each other. In embodiments comprising the rotatable element, the rotatable element may be rotated relative to the container and/or the dosing assembly in order to achieve that the fastening projection is moved in radially out of the depressions.

In one embodiment the one or more of the fastening projection(s) define a second inclined surface adapted to engage a surface of the corresponding fastening depression whereby the fastening projection(s) is/are forced radially inward or outward when the container is rotated relative to the dosing assembly. The second inclined surface may define a normal having a first component being parallel with a tangent to an outer surface of the medical device and a second component extending in a radial direction of the device. Upon rotation of the fastening projection the second inclined surface will abut a surface of the depression whereby the projection is forced inward or outward depending on the design of the medical delivery system. The rotational movement may be a relative rotation between the container and the dosing assembly of less than one revolution, such as less than 180 degrees, such as less than 90 degrees. In a specific embodiment the rotational movement is between 5 and 45 degrees, such as between 15 and 35 degrees, such as 15 degrees, such as 20 degrees, such as 25 degrees, such as 30 degrees.

In order to lock the container to the dosing assembly, each of the first and second fastening means may define a depression. The depressions may be provided on an inner or outer surface of the dosing assembly and the container such that when the container is locked to the dosing assembly, a cavity is defined by the two depressions. In one embodiment the depression of the container is defines on an outer surface and the depression of the dosing assembly is defined on an inner surface, and, thus, when the container is inserted into the dosing assembly, the depressions may aligned so as to define the cavity. In an alternative embodiment the depression of the container is defined on an inner surface of the container and the depression of the dosing assembly is defined on an outer surface of the dosing assembly which is adapted to be inserted into the container.

Moreover, in order to lock the container to the dosing assembly by means of the two depressions, one of the first and second fastening means may further comprise a locking member which is radially movable in the cavity defined by the depressions when the container is fastened to the dosing assembly. When the two depressions are aligned relative to each other and the locking member is positioned in the cavity defined by the two depressions, the container and the dosing assembly is locked against relative translational movement.

Each of the depressions may be defined as a circumferential groove defined on an outer surface of the container or the dosing assembly or as a groove defined in an inner surface of the container or the dosing assembly. Each groove may define at least one surface defining a plane transverse to the longitudinal axis of the device, such as a plane being substantially at right angle of the device. In one embodiment at least one of the grooves defines two substantially plane surfaces each of which is substantially at right angle to the longitudinal axis of the device.

The locking member may be C-shaped or ring-shaped, e.g. by defining a cylinder or a torus. The locking member may comprise two substantially parallel surfaces which during use are at a substantially right angle to the longitudinal axis of the device.

When the locking member is provided in the cavity defined by both the depressions/grooves such that a first part of the ring shaped element is provided in a first of the two depressions and a second part of the ring-shaped element is provided in a second of the two depressions, the container is prevented from being removed from the dosing assembly. Accordingly, the locking member must be moved out of one of the depressions in order to unlock the container from the dosing assembly. Thus, in one embodiment the locking member is adapted to be moved between a first position wherein a first part of the locking member is located in a first of the two depressions/grooves and a second part of the locking member is located in a second of the two depressions/grooves, and a second position wherein a part of the locking member is located in the first of the two depressions/grooved and wherein the locking member is not located in the second of the depressions/grooves.

In order to allow the locking member to be moved between the two positions, the locking member may be adapted to contract or expand radially upon relative rotational movement between the container and the dosing assembly, when the container is fastened to the dosing assembly.

In one embodiment the locking member defines a substantially C-shaped element having two end surfaces which when moved away from each other circumferentially causes the C-shaped element to expand. Accordingly, the container and the dosing assembly may be adapted to force the two end surfaces away from each other circumferentially so as to unlock the container from the dosing assembly. Thus in one embodiment, each of the container and the dosing assembly comprises a radially extending abutment surface e.g. defined by radially extending knobs, each of which engages a corresponding radially extending surface of the locking member such that upon relative rotation between the container and the dosing assembly, the abutment surfaces of the container and the dosing assembly apply an oppositely directed circumferential pressure to the locking member causing each of the radially extending surfaces of the locking member to expand or contract.

The locking member may define one or more curved surfaces facing in a proximal or distal direction of the device. Each of the curved surfaces may define at least two non-parallel normals each of which having a component which is parallel with the longitudinal axis of the device, when the locking member is located in the depressions of the first and second fastening means. Similarly the fastening depression(s) of the container may define curved distal or proximal surfaces, each of which define at least two non-parallel normals having a component which is parallel with the longitudinal axis of the device.

In a SECOND aspect the present invention relates to a container suitable for use (adapted to be used) in a medical delivery system according to the first aspect of the invention.

It will be appreciated that the invention according to the second aspect may comprise any feature and/or element of the invention according to the first aspect. In particular the container of the second aspect may comprise any feature and/or element of the container according to the first aspect of the invention.

Furthermore, the container may be adapted to contain a medicament in a chamber and a slidably arranged piston which is movable in a distal direction towards an outlet so as to reduce the volume of the chamber and expel the medicament. The container may comprise a fastening means defining a circumferentially extending depression and a ring shaped locking member received in the depression, the ring shaped element being radially movable in the depression. The circumferentially extending depression may be provided on an outer surface such that the depression extends inwardly towards a centre axis of the container. Alternatively, the depression may be provided on an inner surface such that the depression extends outwardly and away from a centre axis of the container.

In another embodiment, the container may comprise a fastening means defining a circumferentially extending depression and a radially extending protrusion, the protrusion having a radially extending abutment surface where said radially extending abutment surface is at least partly aligned axially with said circumferentially extending depression. Again, the circumferentially extending depression may be provided on an outer surface such that the depression extends inwardly towards a centre axis of the container, whereby the radially extending protrusion extends outwards away from the centre axis of the container. Alternatively, the depression may be provided on an inner surface such that the depression extends outwardly and away from a centre axis of the container, whereby the radially extending protrusion extends radially inwards towards the centre axis of the container. In both instances, the protrusion may extend from a bottom surface section of the circumferentially extending depression.

Moreover, the container may comprise one or more radially extending coding projections and/or depressions. Additionally, the container may comprise one or more axially extending protrusions and/or indentations.

In a THIRD aspect the present invention relates to a dosing assembly suitable for use (adapted to be used) in a medical delivery system according to the first aspect of the invention.

It will be appreciated that the invention according to the third aspect may comprise any feature and/or element of the invention according to the first aspect. In particular the dosing assembly of the third aspect may comprise any feature and/or element of the dosing assembly according to the first aspect of the invention.

### DESCRIPTION OF THE DRAWINGS

The invention will now be described in detail with reference to the drawings in which:
Figs. 1-5 disclose a first embodiment of a part of the medical delivery system,
Figs. 6-8 disclose a second embodiment of a part of the medical delivery system,
Figs. 9 and 10 each discloses alternatives to details of the first, second and third embodiments disclosed in Figs. 1-8 and Figs. 11-14,
Figs. 11-14 disclose a third embodiment of a part of the medical delivery system, and
Figs. 15-19 disclose a fourth embodiment of a part of the medical delivery system.

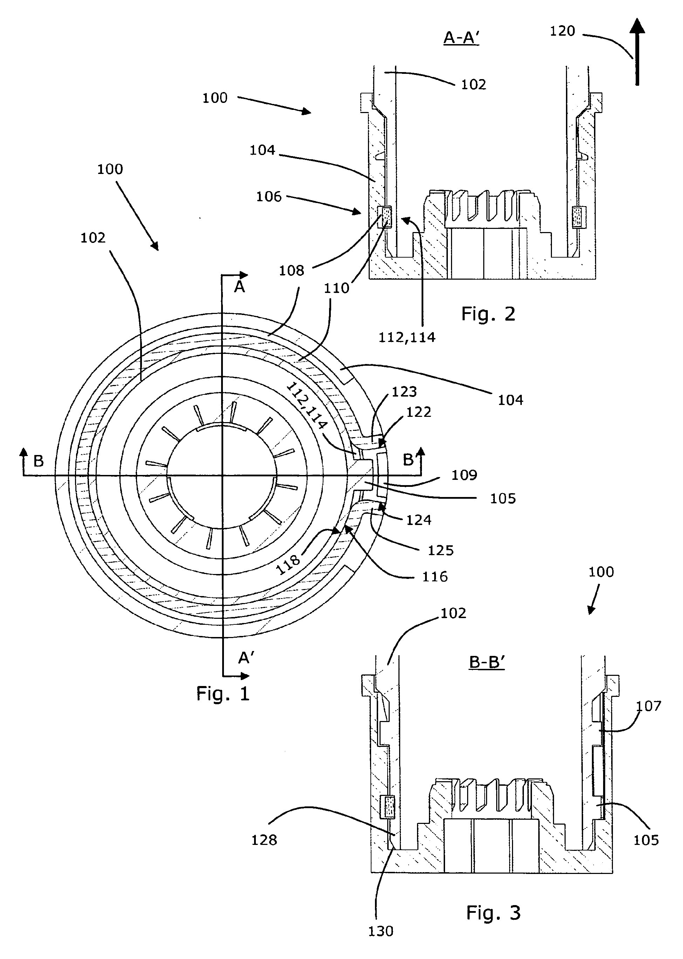

Fig. 1 discloses a part of a medical delivery system 100 comprising a container 102 and a dosing assembly 104. In the drawings only a part of the container 102 and a part of the dosing assembly 104 is illustrated. As an example, the container of the present invention further comprises a chamber (not shown) defined by the container 102 and a slidingly arranged piston (not shown), which chamber is adapted to accommodate a medicament to be injected. Sections of the medical delivery system of Fig. 1 are discloses in Figs. 2 and 3. Fig. 2 discloses section A-A' which neither extends through projection 105 nor through coding projections 107, and Fig. 3 discloses section B-B' which does extend through both projection 105 and coding projection 107.

The dosing assembly 104 comprises a first fastening means 106 defining a groove-shaped depression 108 and comprising a C-shaped rotatable element 110. Moreover, the container 102 comprises a second fastening means 112 also defining a groove-shaped depression 114.

In Fig. 2, a first part of the C-shaped rotatable element 110 is provided in the groove-shaped depression 114 such that a radially inwardly facing surface 116 of the C-shaped rotatable element 110 abuts a radially outwardly facing surface 118 of the groove-shaped depression 114 of the container 102. Furthermore in Fig. 2, a second part of the C-shaped rotatable element 110 is provided in the groove-shaped depression 108 of the dosing assembly. Thus, as the C-shaped rotatable element 110 is provided in both the depressions 108,114, the container is locked axially to the dosing assembly. Accordingly any attempt to move the container 102 axially in the distal direction 120 relative to the dosing assembly 104 causes a distal facing surface of groove-shaped depression 114 of the container 102 to abut a proximal facing surface of the C-shaped rotatable element 110 and a distal facing surface of the C-shaped rotatable element 110 to a abut a proximal facing surface of the dosing assembly 104. The radial width of the rotatable element 110 is smaller than or equal to the radial depth of the groove-shaped depression 108 of the dosing assembly 104, whereby the container 102 may be unlocked from the dosing assembly 104 by moving the C-shaped rotatable element 110 radially outward into the groove-shaped depression 108.

When the radially extending surfaces 122,124 of the C-shaped rotatable element 110 are moved away from each other (circumferentially), the C-shaped rotatable element 110 expands radially. This may be achieved, by rotating the container 102 relative to the dosing assembly 104 about the longitudinal axis of the device, whereby the projection 105 will abut one of the radially extending surfaces 122,124 while the other one of the radially extending surfaces 122,124 abuts a surfaces of the knob 109 of the dosing assembly. Further rotation causes the C-shaped rotatable element 110 to be moved out of the groove-shaped depression 114 of the container 102, whereby the container 102 is unlocked from the dosing assembly 104 and is free to be moved in the distal direction 120.

In the embodiment of Fig. 1, the radially extending surfaces 122,124 are defined on bent end parts 123,125 of the C-shaped rotatable element 110. In other embodiments of the C-shaped element 120, the radially extending surfaces 122,124 are defined by end surfaces of the C-shaped element.

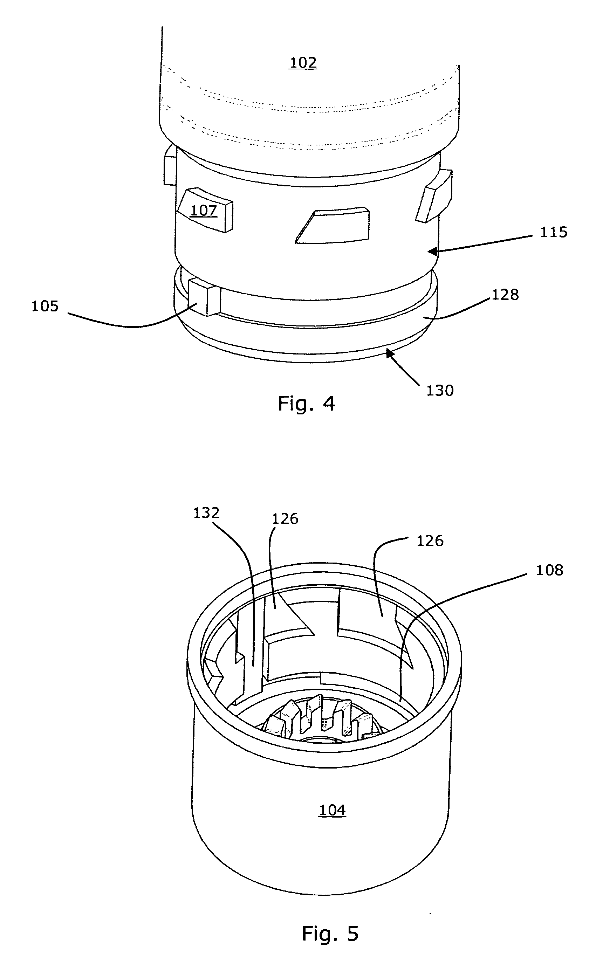

The container 102 and the dosing assembly 104 also comprise radially extending coding projections 107 and radially extending coding depressions 126 as may be seen in Figs. 3, 4 and 5. The coding projections 107 and the coding depressions 126 are provided in a predetermined pattern, such that only containers and dosing assemblies having the same pattern may be attached to each other. This increases safety as a user cannot accidentally attach a container with a wrong medicament to the specific dosing assembly in question. In order to allow the container 102 and the dosing assembly 104 to be rotated relative to each other - when locked to each other - the width of the coding depressions 126 increases in the proximal direction of the dosing assembly 104, cf. Fig. 5.

Furthermore, in order to allow the C-shaped element to be received in both groove-shaped depressions 108,114, a proximal end 128 of the container 102 defines an inclined surface 130, which during insertion of the container 102 into the dosing assembly 104 causes the C-shaped element 110 to expand radially. Upon further axial movement of the container 102 into the dosing assembly 104 the C-shaped element 110 is forced onto an outer surface of the container 102 and finally received in the groove-shaped depression 114 defined on the outer surface 115 of the container 102. During receipt of the C-shaped element 110 in the groove-shaped depression 114 the C-shaped element contracts radially.

In order to allow the projection 105 to be received in the dosing assembly 104, the dosing assembly 104 comprises an axially extending track 132.

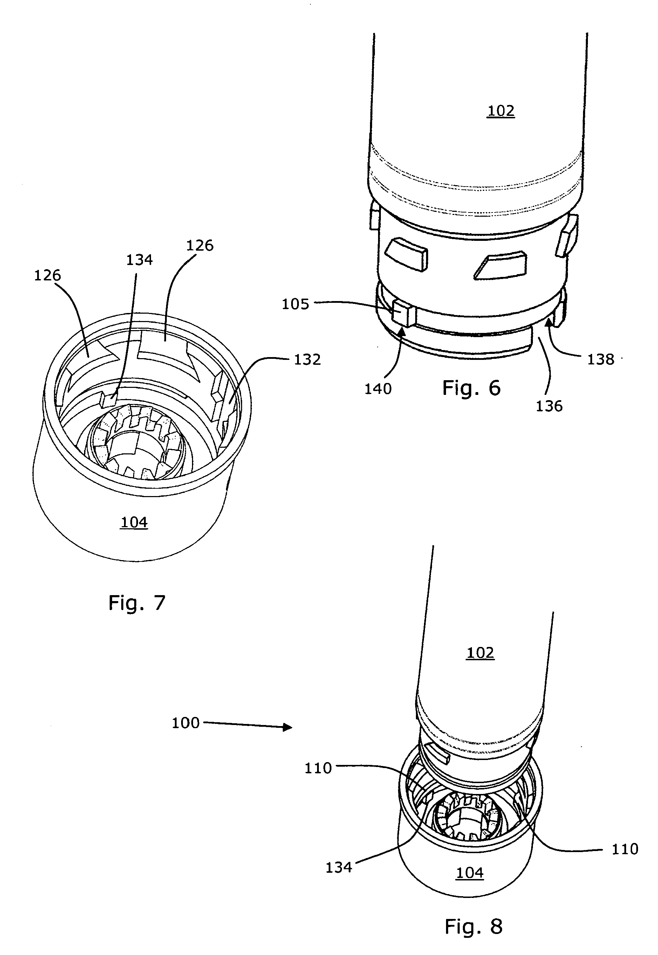

In the embodiment of Figs. 6-8, patient safety is increased even further as the dosing assembly 104 comprises axially extending protrusions 134 and the container 102 comprises axially extending indentations 136. Only containers 102 and dosing assemblies 104 with matching indentations 136 and protrusions 134 may be fastened to each other. In the embodiment of Fig. 6, a proximal facing bottom surface 138 of the indentation 136 and the proximal facing surface 140 of the projection 105 are positioned in the same or substantially the same axial position. However, in some embodiments the surface 140 is positioned proximal relative to the surface 138.

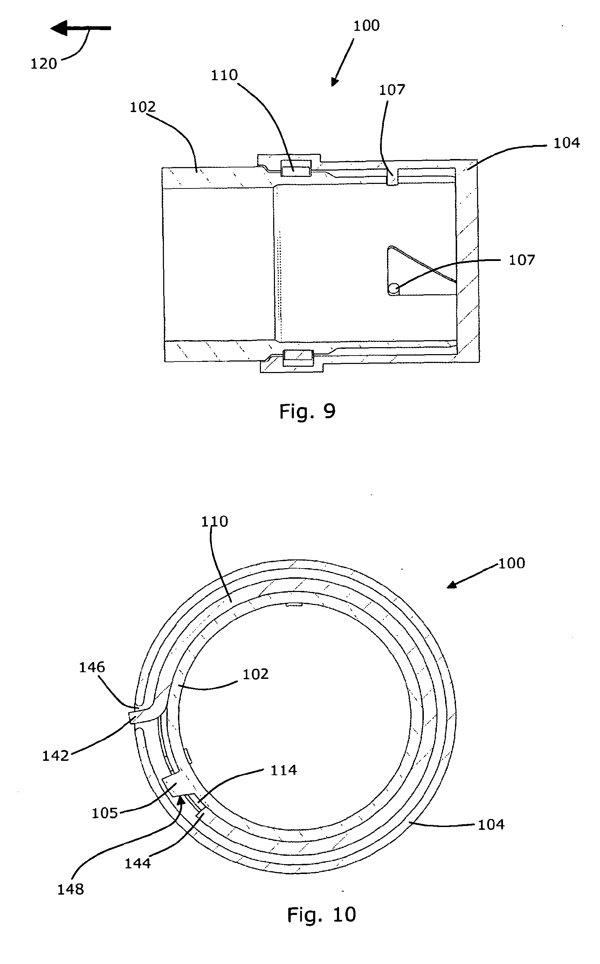

In the alternative disclosed in Fig. 9 the C-shaped rotatable element 110 is positioned distally relative to the radially extending coding projections 107.

In Fig. 10 the C-shaped locking member 110 comprises a bent end 142 and a non-bent end 144. The bent end 142 is received in a passage 146 whereby the bent end 142 is allowed to move radially. However, in some embodiments the bent end 142 is retained in its most radial position. When the container 102 is rotated counter-clockwise relative to the dosing assembly 104, the projection 105 of the container 102 will abut the non-bent end 144 of the C-shaped element 110. This causes a radially extending surface of the bent end 142 to abut a surface of the passage 146 and the projection 105 to abut an end surface of the non-bent end 144, whereby further rotation of the container 102 causes the C-shaped element 110 to expand radially. In order to move the non-bent end 144 out of the groove-shaped depression 114 of the container, the projection 105 comprises an inclined surface 148, which causes the non-bent end 144 to be forced radially outward when the projection 105 is rotated counter-clockwise.

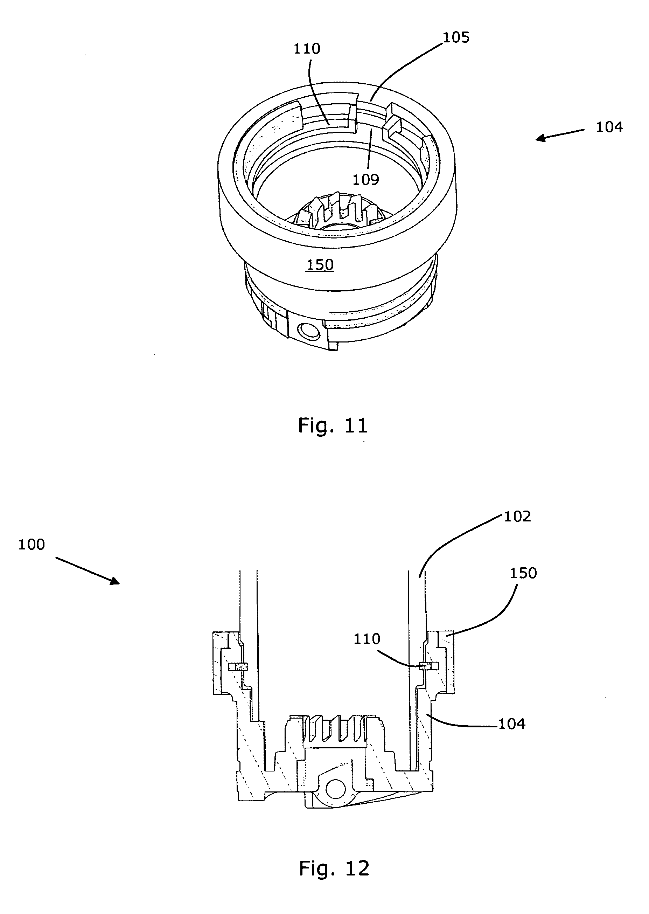

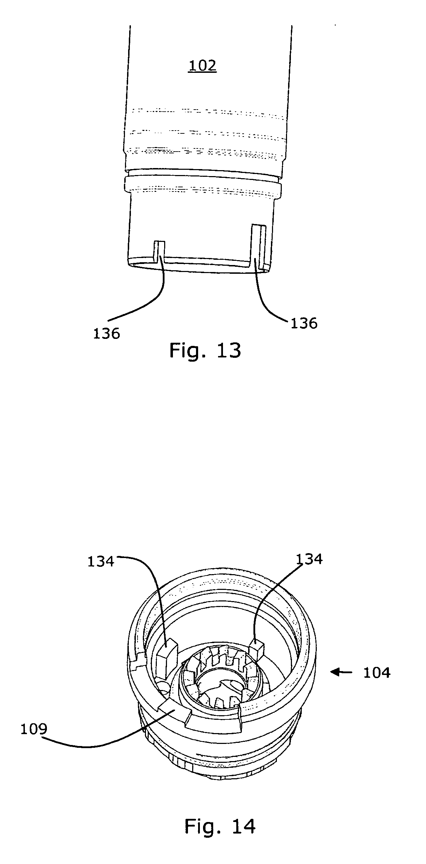

In Figs. 11-14 the dosing assembly 104 comprises a rotatable gripping member 150 which when rotated relative to the dosing assembly 104 causes the C-shaped element 110 to expand. When the projection 105 of the rotatable gripping member 150 is rotated relative to the projection 109 of the dosing assembly 104, the C-shaped element is forced to expand radially whereby the container 102 is unlocked from the dosing assembly 104. In Figs. 13 and 14 the container 102 comprises axially extending indentations 136 which are adapted to receive axially extending protrusions 134 of the dosing assembly 104. The protrusions 134 and the indentations 136 are used to improve patient safety as described above.

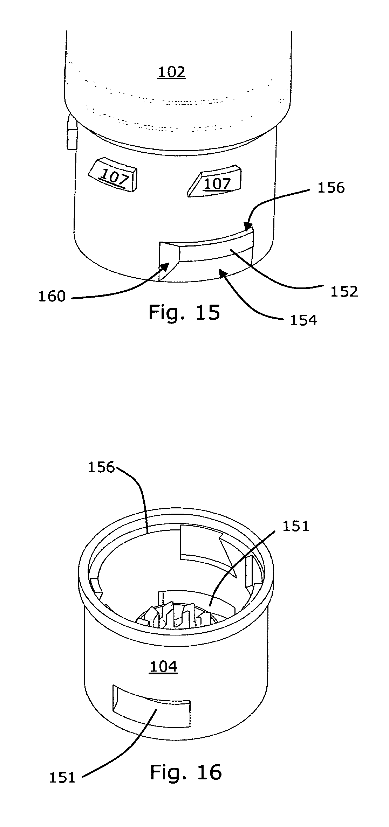

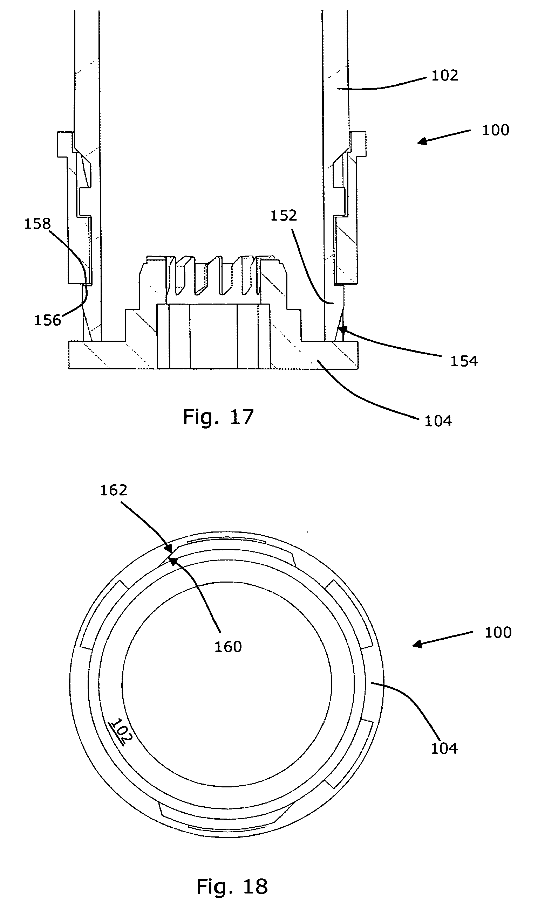

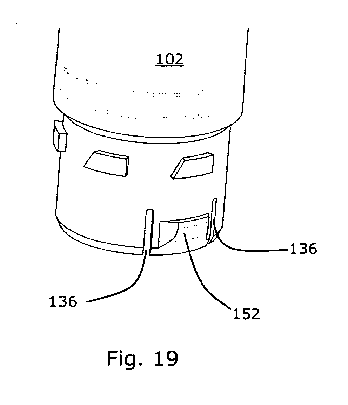

In Figs. 15-19 the first fastening means of the dosing assembly 104 defines a depression 151 adapted to receive fastening projections 152 defined by the second fastening means of the container 102. In the embodiment of Figs. 15-17, the depressions 151 of the dosing assembly 104 extend through a sidewall of the dosing assembly 104, such that the depression 151 define openings on an inner and an outer surface of the sidewall. The fastening projections 152 comprises a first inclined surface 154 which when the container 102 is forced in the dosing assembly 104 abut a rim 156 of the dosing assembly 104, whereby the fastening projections 152 are forced radially inward due to the inclined surface. Upon further relative axial movement between the container 102 and the dosing assembly 104, the fastening projection 152 is received into the depression 151. Due to abutment between the distal facing surface 156 of the projection 152 and the proximal facing surface 158 of the dosing assembly 104, the container 102 is locked to the dosing assembly 104. Upon relative rotation between the container 102 and the dosing assembly 104, the projection 151 is forced radially inward due to engagement between a second inclined surface 160 of the container 102 and the surface 162 of the dosing assembly 104. As shown in Fig. 19 the container may comprises axially extending indentations 136 between which the fastening projections 152 are provided, whereby the force needed to move the projections 152 inwardly may be decreased, as only the material provided between the indentations must be bent in order to move the projections 152 inwardly. The container and dosing assembly of Figs. 15-19 comprises coding projections 107 and axially extending tracks 132 for improving user safety as described in the aforementioned.

## Claims

1. A medical delivery system (100) comprising:
- a container (102) adapted to contain a medicament in a chamber and a slidably arranged piston which is movable in a distal direction towards an outlet so as to reduce the volume of the chamber and expel the medicament;
- a dosing assembly (104) adapted to be fastened to the container (102), so as to allow a driver of the dosing assembly to move the piston of the container in the distal direction;
- wherein the dosing assembly (104) defines a first fastening means (106) which during fastening of the container (102) to the dosing assembly (104) engages a second fastening means (112) of the container (102), **characterized in that** the first and/or second fastening means (106,112) are shaped such that the container (102) is adapted to be:
- locked to the dosing assembly (104) through a translational and non-rotational movement between one of the container (102) and the dosing assembly (104) and at least a part of the other one of the container (102) and the dosing assembly (104), and
- unlocked from the dosing assembly (104) through a rotational and non-translational movement between one of the container (102) and the dosing assembly (104) and at least a part of the other one of the container (102) and the dosing assembly (104).

2. A medical delivery system according to claim 1, wherein one of the container (102) and the dosing assembly (104) comprises one or more radially extending coding projections (107) adapted to be received in corresponding radially extending coding depressions (126) of the other one of the container (102) and the dosing assembly (104) and wherein the container (102) is prevented from being locked to the dosing assembly (104) unless the radially extending coding projections (107) and the radially extending coding depressions (126) define predetermined coding geometries.

3. A medical delivery system according to claim 1 or 2, wherein one of the container (102) and the dosing assembly (104) comprises one or more axially extending protrusions (134) adapted to be received in corresponding indentations (136) in the other one of the container (102) and the dosing assembly (104) and wherein the container (102) is prevented from being locked to the dosing assembly (104) unless the axially extending protrusions (134) and the axially extending indentations (134) define predetermined coding geometries.

4. A medical delivery system according to any of the preceding claims, wherein one of the container (102) and the dosing assembly (104) comprises a rotatable gripping member (150) and wherein the container (102) is adapted to be:
- locked to the dosing assembly (104) through a translational and non-rotational movement between the rotatable gripping member (150) and the other one of the container and the dosing assembly, and
- unlocked from the dosing assembly (104) through a rotational and non-translational movement between the rotatable gripping member (150) and the other one of the container and the dosing assembly.

5. A medical delivery system according to any of claims 1-4, wherein one of the first and second fastening means (106,112) defines at least one radially extending fastening projection (152) adapted to be received in a corresponding radially extending fastening depression (151) of the other one of the first and second fastening means (106,112).

6. A medical delivery system according to claim 5, wherein respective ones of the at least one fastening projection (152) define a respective first inclined surface (154) for forcing the fastening projection (152) radially inward or outward when the container (102) is brought into engagement with the dosing assembly (104) during attachment of the container (102) to the dosing assembly (104).

7. A medical delivery system according to any of claims 5 or 6, wherein respective ones of the at least one fastening projection (152) define a second inclined surface (160) adapted to engage a surface (162) of the corresponding fastening depression (151) whereby the fastening projection (152) is forced radially inward or outward when the container (102) is rotated relative to the dosing assembly (104).

8. A medical delivery system according to any of claims 1-4, wherein each of the first and second fastening means (106,112) defines a depression (108,114), said depressions being axially aligned when the container (102) is fastened to the dosing assembly (104), and wherein one of the first and second fastening means (106,112) further comprises a locking member (110) which is radially movable in a cavity defined by the depressions (108,114) when the container (102) is fastened to the dosing assembly (104).

9. A medical delivery system according to claim 8 when dependent on any of the claims 1-3, wherein the locking member (110) is adapted to contract or expand radially upon relative rotational movement between the container (102) and the dosing assembly (104) when the container (102) is fastened to the dosing assembly (104), so as to unlock the container (102) from the dosing assembly (104), whereby the container (102) may be moved translationally relative to the dosing assembly (104).

10. A medical delivery system according to claim 9, wherein each of the container (102) and the dosing assembly (104) comprises a radially extending abutment surface (146,148) each of which engages a corresponding radially extending surface (142,144) of the locking member (110) such that upon relative rotation between the container (102) and the dosing assembly (104), the abutment surfaces (146,148) apply a circumferential pressure to the locking member (110) causing the locking member (110) to expand or contract.

11. A container for use in a medical delivery system (100) according to any of claims 1-10, the container (102) being adapted to contain a medicament in a chamber and a slidably arranged piston which is movable in a distal direction towards an outlet so as to reduce the volume of the chamber and expel the medicament.

12. A container according to claim 11 for use in a medical delivery system (100) according to any of claims 8-10, the container (102) comprising a fastening means (112) defining a circumferentially extending depression (114) and a ring shaped locking member (110) received in the depression (114), the ring shaped member (110) being radially movable in the depression (114).

13. A container according to claim 11 for use in a medical delivery system (100) according to any of claims 8-10, the container (102) comprising a fastening means (112) defining a circumferentially extending depression (114) and radially extending protrusion (105) having a radially extending abutment surface (148), said radially extending abutment surface (148) being at least partly aligned axially with said circumferentially extending depression (114).

14. A container according to claim 11 for use in a medical delivery system (100) according to claim 7, the container (102) comprising a fastening means (112) defining at least one radially extending fastening projection (152) adapted to be received in a corresponding radially extending fastening depression (151) of a dosing assembly (104), each of said at least one fastening projections (152) further defining:
- a first inclined surface (154) for forcing the fastening projection (152) radially inward or outward when the container (102) is brought into engagement with the dosing assembly (104) by a translational and non-rotational movement during attachment of the container (102) to the dosing assembly (104), and
a second inclined surface (160) adapted to engage a surface (162) of the corresponding fastening depression (151) whereby the fastening projection (152) is forced radially inward or outward when the container (102) is rotated relative to the dosing assembly (104).

15. A container according to any of claims 11-14, wherein the container (102) further comprises one or more radially extending coding projections (107)/depressions and/or wherein the container comprises one or more axially extending protrusions/indentations (136).

16. A dosing assembly suitable for use in a medical delivery system (100) according to any of claims 1-10.

## Patentansprüche

1. Medikamentenabgabesystem (100), umfassend:
- einen Behälter (102), der angepasst ist, ein Medikament in einer Kammer und einen verschiebbar angeordneten Kolben, der in distaler Richtung zu einem Auslass hin derart beweglich ist, dass er das Volumen der Kammer verringert und das Medikament ausstößt, aufzunehmen;
- eine Dosieranordnung (104), die angepasst ist, an dem Behälter (102) derart befestigt zu werden, dass es einem Antriebsmittel der Dosieranordnung ermöglicht wird, den Kolben des Behälters in distaler Richtung zu bewegen;
- wobei die Dosieranordnung (104) ein erstes Befestigungsmittel (106) definiert, das während des Befestigens des Behälters (102) an der Dosieranordnung (104) in ein zweites Befestigungsmittel (112) des Behälters (102) eingreift, **dadurch gekennzeichnet, dass** das erste und/oder das zweite Befestigungsmittel (106, 112) derart geformt sind, dass der Behälter (102) derart angepasst ist, dass er
- an der Dosieranordnung (104) durch eine translatorische und nicht-drehende Bewegung zwischen Einem des Behälters (102) und der Dosieranordnung (104) und mindestens einem Teil des Anderen des Behälters (102) und der Dosieranordnung (104) arretiert wird, und
- von der Dosieranordnung (104) durch eine drehende und nicht-translatorische Bewegung zwischen Einem des Behälters (102) und der Dosieranordnung (104) und mindestens einem Teil des Anderen des Behälters (102) und der Dosieranordnung (104) entriegelt wird.

2. Medikamentenabgabesystem nach Anspruch 1, wobei Einer des Behälters (102) und der Dosieranordnung (104) einen oder mehrere sich radial erstreckende Kodiervorsprünge (107) umfasst, die angepasst sind, in entsprechenden sich radial erstreckenden Kodiervertiefungen (126) des Anderen des Behälters (102) und der Dosieranordnung (104) aufgenommen zu werden, und wobei verhindert wird, dass der Behälter (102) an der Dosieranordnung (104) arretiert wird, wenn die sich radial erstreckenden Kodiervorsprünge (107) und die sich radial erstreckenden Kodiervertiefungen (126) nicht vorbestimmte Kodiergeometrien definieren.

3. Medikamentenabgabesystem nach Anspruch 1 oder 2, wobei Einer des Behälters (102 und der Dosieranordnung (104) einen oder mehrere sich axial erstreckende Ausbuchtungen (134) umfasst, die angepasst sind, in entsprechenden Einbuchtungen (136) in dem Anderen des Behälters (102) und der Dosieranordnung (104) aufgenommen zu werden, und wobei verhindert wird, dass der Behälter (102) an der Dosieranordnung (104) arretiert wird, wenn die sich axial erstreckenden Ausbuchtungen (134) und die sich axial erstreckenden Einbuchtungen (136) nicht vorbestimmte Kodiergeometrien definieren.

4. Medikamentenabgabesystem nach einem der vorangehenden Ansprüche, wobei Einer des Behälters (102) und der Dosieranordnung (104) ein drehbares Greifelement (150) umfasst und wobei der Behälter (102) derart angepasst ist, dass er
- an der Dosieranordnung (104) durch eine translatorische und nicht-drehende Bewegung zwischen dem drehbaren Greifelement (150) und dem Anderen des Behälters und der Dosieranordnung arretiert wird, und
- von der Dosieranordnung (104) durch eine drehende und nicht-translatorische Bewegung zwischen dem drehbaren Greifelement (150) und dem Anderen des Behälters und der Dosieranordnung entriegelt wird.

5. Medikamentenabgabesystem nach einem der Ansprüche 1-4, wobei Eines des ersten und des zweiten Befestigungsmittel (106, 112) mindestens einen sich radial erstreckenden Befestigungsvorsprung (152) definiert, der angepasst ist, in einer entsprechenden sich radial erstreckenden Befestigungsvertiefung (151) des Anderen des ersten und des zweiten Befestigungsmittels (106, 112) aufgenommen zu werden.

6. Medikamentenabgabesystem nach Anspruch 5, wobei Jeweilige des mindestens einen Befestigungsvorsprungs (152) eine jeweilige erste geneigte Oberfläche (154) definieren, um den Befestigungsvorsprung (152) radial nach innen oder nach außen zu zwängen, wenn der Behälter (102) mit der Dosieranordnung (104) während des Anbringens des Behälters (102) an der Dosieranordnung (104) in Eingriff gebracht wird.

7. Medikamentenabgabesystem nach einem der Ansprüche 5 oder 6, wobei Jeweilige des mindestens einen Befestigungsvorsprungs (152) eine zweite geneigte Oberfläche (160) definieren, die angepasst ist, in eine Oberfläche (162) der entsprechenden Befestigungsvertiefung (151) einzugreifen, wodurch der Befestigungsvorsprung (152) radial nach innen oder nach außen gezwängt wird, wenn der Behälter (102) in Bezug auf die Dosieranordnung (104) gedreht wird.

8. Medikamentenabgabesystem nach einem der Ansprüche 1-4, wobei Jedes des ersten und des zweiten Befestigungsmittels (106, 112) eine Vertiefung (108, 114) definiert, wobei die Vertiefungen axial ausgerichtet sind, wenn der Behälter (102) an der Dosieranordnung (104) befestigt ist, und wobei Eines des ersten und des zweiten Befestigungsmittels (106, 112) des Weiteren ein Arretierelement (110) umfasst, das in einem durch die Vertiefungen (108, 114) definierten Hohlraum radial beweglich ist, wenn der Behälter (102) an der Dosieranordnung (104) befestigt ist.

9. Medikamentenabgabesystem nach Anspruch 8, falls abhängig von einen der Ansprüche 1-3, wobei das Arretierelement (110) so angepasst ist, um sich bei relativer drehender Bewegung zwischen dem Behälter (102) und der Dosieranordnung (104) derart einzuziehen oder zu erweitern, wenn der Behälter (102) an der Dosieranordnung (104) befestigt ist, dass der Behälter (102) von der Dosieranordnung (104) entriegelt wird, wodurch der Behälter (102) translatorisch in Bezug auf die Dosieranordnung (104) bewegt werden kann.

10. Medikamentenabgabesystem nach Anspruch 9, wobei Jedes des Behälters (102) und der Dosieranordnung (104) eine sich radial erstreckende Anlagefläche (146, 148) umfasst, wobei Jede davon derart in eine entsprechende sich radial erstreckende Oberfläche (142, 144) des Arretierelements (110) eingreift, dass die Anlageflächen (146, 148) bei relativer Drehung zwischen dem Behälter (102) und der Dosieranordnung (104) auf das Arretierelement (110) einen umlaufenden Druck ausüben, was bewirkt, dass sich das Arretierelement (110) erweitert oder einzieht.

11. Behälter zur Verwendung in einem Medikamentenabgabesystem (100) nach einem der Ansprüche 1-10, wobei der Behälter (102) angepasst ist, ein Medikament in einer Kammer und einen verschiebbar angeordneten Kolben, der in distaler Richtung zu einem Auslass hin derart beweglich ist, dass er das Volumen der Kammer verringert und das Medikament ausstößt, aufzunehmen.

12. Behälter nach Anspruch 11 zur Verwendung in einem Medikamentenabgabesystem (100) nach einem der Ansprüche 8-10, wobei der Behälter (102) ein eine sich umlaufend erstreckende Vertiefung (114) definierendes Befestigungsmittel (112) und ein in der Vertiefung (114) aufgenommenes ringförmiges Arretierelement (110) umfasst, wobei das ringförmige Element (110) in der Vertiefung (114) radial beweglich ist.

13. Behälter nach Anspruch 11 zur Verwendung in einem Medikamentenabgabesystem (100) nach einem der Ansprüche 8-10, wobei der Behälter (102) ein eine sich umlaufend erstreckende Vertiefung (114) definierendes Befestigungsmittel (112) und eine sich radial erstreckende Einbuchtung (105) mit einer sich radial erstreckenden Anlagefläche (148) umfasst, wobei die sich radial erstreckende Anlagefläche (148) zumindest teilweise mit der sich umlaufend erstreckenden Vertiefung (114) ausgerichtet ist.

14. Behälter nach Anspruch 11 zur Verwendung in einem Medikamentenabgabesystem (100) nach Anspruch 7, wobei der Behälter (102) ein Befestigungsmittel (112) umfasst, das zumindest einen sich radial erstreckenden Befestigungsvorsprung (152) definiert, der angepasst ist, in einer entsprechenden sich radial erstreckenden Befestigungsvertiefung (151) einer Dosieranordnung (104) aufgenommen zu werden, wobei jeder der mindestens einen Befestigungsvorsprünge (152) des Weiteren Folgendes definiert:
- eine erste geneigte Oberfläche (154), um den Befestigungsvorsprung (152) radial nach innen oder nach außen zu zwängen, wenn der Behälter (102) mit der Dosieranordnung (104) während des Anbringens des Behälters (102) an der Dosieranordnung (104) durch eine translatorische und nicht drehende Bewegung in Verbindung gebracht wird, und
- eine zweite geneigte Oberfläche (160), die angepasst ist, in eine Oberfläche (162) der entsprechenden Befestigungsvertiefung (151) einzugreifen, wodurch der Befestigungsvorsprung (152) radial nach innen oder nach außen gezwängt wird, wenn der Behälter (102) in Bezug auf die Dosieranordnung (104) gedreht wird.

15. Behälter nach einem der Ansprüche 11-14, wobei der Behälter (102) des Weiteren eine(n) oder mehrere sich radial erstreckende Kodiervorsprünge (107)/-vertiefungen umfasst und/oder wobei der Behälter eine(n) oder mehrere sich axial erstreckende Ausbuchtungen/Einbuchtungen (136) umfasst.

16. Dosieranordnung, die zur Verwendung in einem Medikamentenabgabesystem (100) nach einem der Ansprüche 1-10 geeignet ist.

## Revendications

1. Un système médical de délivrance (100), comprenant :
- un conteneur (102) apte à contenir un médicament dans une chambre et un piston configuré à coulissement qui est mobile dans une direction distale en direction d'une sortie de manière à réduire le volume de la chambre et expulser le médicament ;
- un ensemble de dosage (104) apte à être solidarisé au conteneur (102), afin de permettre à un entraîneur de l'ensemble de dosage de déplacer le piston du conteneur dans la direction distale ;
- dans lequel l'ensemble de dosage (104) définit un premier moyen de solidarisation (106) qui durant la solidarisation du conteneur (102) à l'ensemble de dosage (104) vient en prise avec un second moyen de solidarisation (112) du conteneur (102), **caractérisé en ce que** le premier et/ou le second moyen de solidarisation (106, 112) sont conformés de telle sorte que le conteneur (102) soit apte à être :
verrouillé à l'ensemble de dosage (104) par l'intermédiaire d'un mouvement de translation et non de rotation entre l'un d'entre le conteneur (102) et l'ensemble de dosage (104) et au moins une partie de l'autre d'entre le conteneur (102) et l'ensemble de dosage (104), et
• déverrouillé de l'ensemble de dosage (104) par l'intermédiaire d'un mouvement de rotation et non de translation entre l'un d'entre le conteneur (102) et l'ensemble de dosage (104) et au moins une partie de l'autre d'entre le conteneur (102) et l'ensemble de dosage (104).

2. Un système médical de délivrance selon la revendication 1, dans lequel l'un d'entre le conteneur (102) et l'ensemble de dosage (104) comprend une ou plusieurs saillies de codage s'étendant radialement (107) aptes à être reçues dans des creux de codage s'étendant radialement correspondants (126) de l'autre d'entre le conteneur (102) et l'ensemble de dosage (104) et dans lequel le conteneur (102) est empêché d'être verrouillé à l'ensemble de dosage (104) à moins que les saillies de codage s'étendant radialement (107) et les creux de codage s'étendant radialement correspondants (126) ne définissent des géométries de codage prédéterminées.

3. Un système médical de délivrance selon la revendication 1 ou 2, dans lequel l'un d'entre le conteneur (102) et l'ensemble de dosage (104) comprend une ou plusieurs saillies s'étendant axialement (134) aptes à être reçues dans des indentations correspondantes (136) dans l'autre d'entre le conteneur (102) et l'ensemble de dosage (104) et dans lequel le conteneur (102) est empêché d'être verrouillé à l'ensemble de dosage (104) à moins que les saillies s'étendant axialement (134) et les indentations s'étendant axialement (136) ne définissent des géométries de codage prédéterminées.

4. Un système médical de délivrance selon l'une des revendications précédentes, dans lequel l'un d'entre le conteneur (102) et l'ensemble de dosage (104) comprend un organe d'agrippement rotatif (150) et dans lequel le conteneur (102) est apte à être :
• verrouillé à l'ensemble de dosage (104) par l'intermédiaire d'un mouvement de translation et non de rotation entre l'organe d'agrippement rotatif (150) et l'autre d'entre le conteneur et l'ensemble de dosage, et
• déverrouillé de l'ensemble de dosage (104) par l'intermédiaire d'un mouvement de rotation et non de translation entre l'organe d'agrippement rotatif (150) et l'autre d'entre le conteneur et l'ensemble de dosage.

5. Un système médical de délivrance selon l'une des revendications 1 à 4, dans lequel l'un d'entre le premier et le second moyen de solidarisation (106, 112) définit au moins une saillie de solidarisation s'étendant radialement (152) apte à être reçue dans un creux correspondant de solidarisation s'étendant radialement (151) de l'autre d'entre le premier et le second moyen de solidarisation (106, 112).

6. Un dispositif de délivrance médicale selon la revendication 5, dans lequel des saillies respectives des au moins une saillie de solidarisation (152) définissent une première surface inclinée respective (154) pour forcer la saillie de solidarisation (152) radialement vers l'intérieur ou vers l'extérieur lorsque le conteneur (102) est amené en contact avec l'ensemble de dosage (104) durant la solidarisation du conteneur (102) à l'ensemble de dosage (104).

7. Un système médical de délivrance selon l'une des revendications 5 ou 6, dans lequel des saillies respectives des au moins une saillie de solidarisation (152) définissent une seconde surface inclinée (160) apte à venir en prise avec une surface (162) du creux de solidarisation correspondant (151) de sorte que la saillie de solidarisation (152) soit forcée radialement vers l'intérieur ou vers l'extérieur lorsque le conteneur (102) est tourné par rapport à l'ensemble de dosage (104).

8. Un système médical de délivrance selon l'une des revendications 1 à 4, dans lequel chacun d'entre le premier et le second moyen de solidarisation (106, 112) définit un creux (108, 114), lesdits creux étant axialement alignés lorsque le conteneur (102) est solidarisé à l'ensemble de dosage (104), et dans lequel l'un d'entre le premier et le second moyen de solidarisation (106, 112) comprend en outre un organe de verrouillage (110) qui est radialement mobile dans une cavité définie par les creux (108, 114) lorsque le conteneur (102) est solidarisé à l'ensemble de dosage (104).

9. Un système médical de délivrance selon la revendication 8 prise en dépendance de l'une des revendications 1 à 3, dans lequel l'organe de verrouillage (110) est apte à se contracter ou à se déployer radialement sous l'effet d'un mouvement de rotation entre le conteneur (102) et l'ensemble de dosage (104) lorsque le conteneur (102) est fixé à l'ensemble de dosage (104), de manière à déverrouiller le conteneur (102) de l'ensemble de dosage (104), de sorte que le conteneur (102) puisse être déplacé en translation par rapport à 1'104.

10. Un système médical de délivrance selon la revendication 9, dans lequel chacun d'entre le conteneur (102) et l'ensemble de dosage (104) comprend une surface de venue en butée s'étendant radialement (146, 148) dont chacune vient en prise avec une surface correspondante s'étendant radialement (142, 144) de l'organe de verrouillage (110) de sorte que sous l'effet d'un mouvement de rotation relatif entre le conteneur (102) et l'ensemble de dosage (104), les surfaces de venue en butée (146, 148) appliquent une pression circonférentielle à l'organe de verrouillage (110) faisant en sorte que l'organe de verrouillage (110) se déploie ou se contracte.

11. Un conteneur destiné à être utilisé dans un système médical de délivrance (100) selon l'une des revendications 1 à 10, le conteneur étant apte à contenir un médicament dans une chambre et un piston configuré à coulissement qui est mobile dans une direction distale en direction d'une sortie de manière à réduire le volume de la chambre et expulser le médicament.

12. Un conteneur selon la revendication 11 pour une utilisation dans un système médical de délivrance (100) selon l'une des revendications 8 à 10, le conteneur (102) comprenant un moyen de solidarisation (112) définissant un creux s'étendant circonférentiellement (114) et un organe de verrouillage en forme d'anneau (110) logé dans le creux (114), l'organe en forme d'anneau (110) étant mobile radialement dans le creux (114).

13. Un conteneur selon la revendication 11 pour une utilisation dans un système médical de délivrance (100) selon l'une des revendications 8 à 10, le conteneur (102) comprenant un moyen de solidarisation (112) définissant un creux s'étendant circonférentiellement (114) et une saillie s'étendant radialement (105) avec une surface de venue en butée s'étendant radialement (148), ladite surface de venue en butée s'étendant radialement (148) étant au moins en partie alignée axialement avec ledit creux s'étendant circonférentiellement (114).

14. Un conteneur selon la revendication 11 pour une utilisation dans un système médical de délivrance (100) selon la revendication 7, le conteneur (102) comprenant un moyen de solidarisation (112) définissant au moins une saillie de solidarisation s'étendant radialement (152) apte à être reçue dans un creux de solidarisation s'étendant radialement correspondant (151) d'un ensemble de dosage (104), chacune d'entre lesdites au moins deux saillies de solidarisation (152) comprenant en outre :
- une première surface inclinée (154) pour forcer la saillie de solidarisation (152) radialement vers l'intérieur ou vers l'extérieur lorsque le conteneur (102) est amené en contact avec l'ensemble de dosage (104) par un mouvement de translation et non de rotation durant la solidarisation du conteneur (102) à l'ensemble de dosage (104), et
- une seconde surface inclinée (160) apte à venir en prise avec une surface (162) du creux de solidarisation correspondant (151) de sorte que la saillie de solidarisation (152) soit forcée radialement vers l'intérieur ou vers l'extérieur lorsque le conteneur (102) est tourné par rapport à l'ensemble de dosage (104).

15. Un conteneur selon l'une des revendications 11 à 14, dans lequel le conteneur (102) comprend en outre une ou plusieurs saillies de codage s'étendant radialement (107) ou creux et/ou dans lequel le conteneur comprend une ou plusieurs saillies ou indentations s'étendant axialement (136).

16. Un ensemble de dosage convenant à une utilisation d'un système médical de délivrance (100) selon l'une des revendications 1 à 10.
